(19) Europäisches Patentamt
European Patent Office
Office européen des brevets

(11) Veröffentlichungsnummer: **0 023 614**
**B1**

(12) # EUROPÄISCHE PATENTSCHRIFT

(45) Veröffentlichungstag der Patentschrift: **02.03.83**

(51) Int. Cl.³: **A 61 K 31/415** // C07D233/60, A01N43/50

(21) Anmeldenummer: **80104038.7**

(22) Anmeldetag: **12.07.80**

(54) **Antimykotische Mittel, die Imidazolylenolether umfassen, ihre Herstellung und ihre Verwendung.**

(30) Priorität: **04.08.79 DE 2931778**

(43) Veröffentlichungstag der Anmeldung: **11.02.81 Patentblatt 81/6**

(45) Bekanntmachung des Hinweises auf die Patenterteilung: **02.03.83 Patentblatt 83/9**

(84) Benannte Vertragsstaaten: **AT BE CH DE FR GB IT LI NL SE**

(56) Entgegenhaltungen: **DE-A-2 720 868**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Draber, Wilfried, Dr., In den Birken 81, D-5600 Wuppertal-1 (DE)**
Erfinder: **Büchel, Karl Heinz, Prof. Dr, Bergerheide 62, D-5600 Wuppertal (DE)**
Erfinder: **Plempel, Manfred, Dr., Pahlkestrasse 5, D-5600 Wuppertal-1 (DE)**
Erfinder: **Haller, Ingo, Dr., Viktoriastrasse 99, D-5600 Wuppertal-1 (DE)**

**EP 0 023 614 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Antimykotische Mittel, die Imidazolylenolether umfassen, ihre Herstellung und ihre Verwendung

Die Erfindung betrifft Antimykotika, die als Wirkstoff mindestens einen Imidazolyl-enolether der allgemeinen Formel I enthalten.

Es ist bereits bekannt geworden, daß Imidazolyl-ether, wie beispielsweise substituierte 3,3-Dimethyl-1-(imidazol-1-yl)-1-phenoxy-2-(R-oxy)-butane, gute antimykotische Eigenschaften aufweisen (vgl. DE-OS 2 720 868). Die Wirkung dieser Verbindungen ist jedoch nicht immer ganz befriedigend.

Es wurde gefunden, daß die neuen Imidazolyl-enolether der allgemeinen Formel I

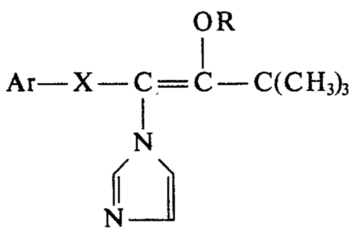

(I)

in welcher

Ar für gegebenenfalls einfach oder zweifach durch Halogen, Alkyl mit 1–3 Kohlenstoffatomen, Trifluormethyl, Nitro, Phenyl oder Chlorphenyl substituiertes Phenyl steht,
R für Alkyl mit 1–4 Kohlenstoffatomen steht und
X für Sauerstoff oder Methylen steht,

und deren physiologisch verträglichen Säureadditions-Salze und Metallsalz-Komplexe gute antimikrobielle, insbesondere antimykotische Eigenschaften aufweisen.

Die erfindungsgemäß zu verwendenden Verbindungen der allgemeinen Formel I kommen in den geometrischen Isomeren E(trans) und Z(cis) vor. Bei der E,Z-Nomenklatur werden die an der Doppelbindung stehenden Substituenten nach der Cahn-Ingold-Prelog-Regel nach abnehmender Priorität eingeordnet. Stehen die bevorzugten Substituenten auf derselben Seite der Doppelbindung, ist die Konfiguration Z (abgeleitet von zusammen), stehen sie auf entgegengesetzter Seite, E (abgeleitet von entgegen). Sowohl die einzelnen Isomeren als auch die Gemische werden erfindungsgemäß beansprucht.

Überraschenderweise zeigen die erfindungsgemäß verwendbaren Imidazolyl-enolether der allgemeinen Formel I eine bessere antimykotische Wirksamkeit als die aus dem Stand der Technik bekannten Imidazolylether, die chemisch und wirkungsmäßig naheliegende Verbindungen sind. Die erfindungsgemäß verwendbaren Wirkstoffe stellen somit eine Bereicherung der Pharmazie dar.

Besonders bevorzugt sind diejenigen Imidazolyl-enolether der allgemeinen Formel I, in denen Ar für Phenyl steht, das gegebenenfalls einfach oder zweifach, gleich oder verschieden substituiert ist durch Fluor, Chlor, Brom, Jod, Methyl, Ethyl, Isopropyl, Trifluormethyl, Nitro, Phenyl oder Chlorphenyl; R für Methyl, Ethyl, Isopropyl, Isobutyl oder tert.-Butyl steht; und X die in der Erfindungsdefinition angegebene Bedeutung hat.

Im einzelnen seien außer den bei den Herstellungsbeispielen genannten Verbindungen die folgenden Verbindungen der allgemeinen Formel I genannt:

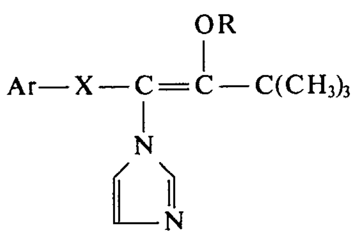

(I)

| Ar | X | R |
|---|---|---|
| 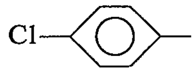 | O | $CH_3$ |
| 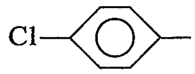 | O | i-$C_3H_7$ |
| 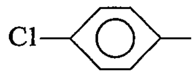 | O | i-$C_4H_9$ |
| 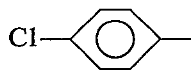 | $CH_2$ | $CH_3$ |
| 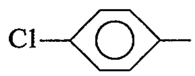 | $CH_2$ | $C_2H_5$ |
| 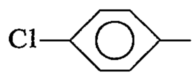 | $CH_2$ | i-$C_3H_7$ |
| 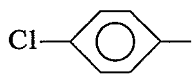 | $CH_2$ | i-$C_4H_9$ |
| 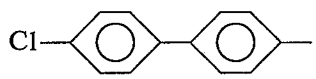 | $CH_2$ | $CH_3$ |
| 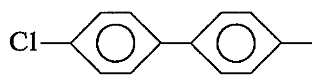 | $CH_2$ | $C_2H_5$ |
| 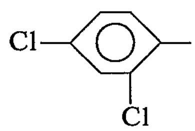 | O | $CH_3$ |
| 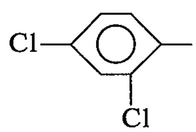 | O | $C_2H_5$ |
| 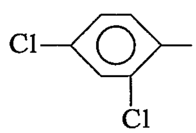 | $CH_2$ | $CH_3$ |
| 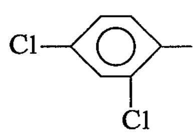 | $CH_2$ | $C_2H_5$ |
| 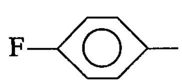 | O | $CH_3$ |
| 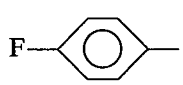 | O | $C_2H_5$ |
| 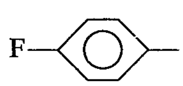 | $CH_2$ | $CH_3$ |
| 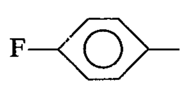 | $CH_2$ | $C_2H_5$ |

Fortsetzung

| Ar | X | R |
|---|---|---|
| 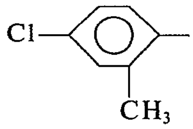 | O | $CH_3$ |
| 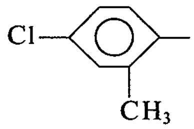 | O | $C_2H_5$ |
| 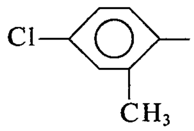 | $CH_2$ | $CH_3$ |
| 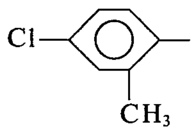 | $CH_2$ | $C_2H_5$ |
| 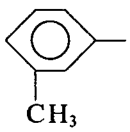 | O | $CH_3$ |
| 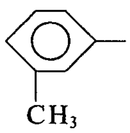 | O | $C_2H_5$ |
| 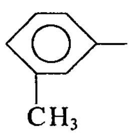 | $CH_2$ | $CH_3$ |
| 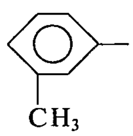 | $CH_2$ | $C_2H_5$ |
| 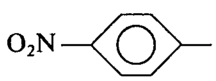 | O | $CH_3$ |
| 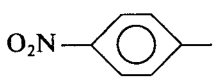 | O | $C_2H_5$ |
| 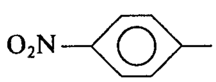 | $CH_2$ | $CH_3$ |
| 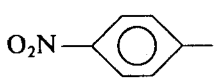 | $CH_2$ | $C_2H_5$ |
| 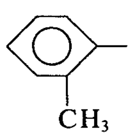 | O | $CH_3$ |

Fortsetzung

| Ar | X | R |
|---|---|---|
| 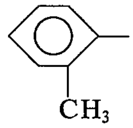 | O | $C_2H_5$ |
| 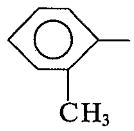 | $CH_3$ | $CH_3$ |
| 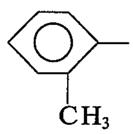 | $CH_3$ | $C_2H_5$ |
| 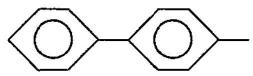 | O | $CH_3$ |
| 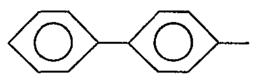 | O | $C_2H_5$ |
| 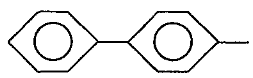 | $CH_2$ | $CH_3$ |
| 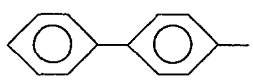 | $CH_2$ | $C_2H_5$ |

Die erfindungsgemäß zu verwendenden Wirkstoffe, deren Säureadditions-Salze und Metallsalz-Komplexe sind noch nicht bekannt. Sie können jedoch gemäß einem eigenen Vorschlag hergestellt werden, indem man Imidazolylazolyl-ketone der allgemeinen Formel II

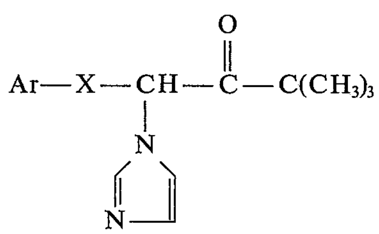 (II)

in welcher

Ar und X die oben angegebene Bedeutung haben,

mit entsprechenden Alkylsulfaten, wie z. B. Dimethyl- oder Diethylsulfat bzw. Alkylhalogeniden, wie insbesondere Alkylbromiden und -jodiden, in Gegenwart eines inerten organischen Lösungsmittels, wie beispielsweise Dimethylsulfoxid, bei Temperaturen zwischen 0 und 100°C umsetzt.

In einer bevorzugten Ausführungsform wird diese Umsetzung in einem Zweiphasensystem, wie beispielsweise wäßrige Natron- oder Kalilauge/Toluol oder Methylenchlorid, gegebenenfalls unter Zusatz von 0,1—1 Mol eines Phasentransfer-Katalysators, wie beispielsweise Ammonium- oder Phosphoniumverbindungen, beispielsweise seien Benzyldodecyl-dimethyl-ammoniumchlorid (Zephirol) und Triethyl-benzyl-ammoniumchlorid genannt, durchgeführt (vergleiche auch die Herstellungsbeispiele).

Die Verbindungen der allgemeinen Formel I fallen in Form der geometrischen Isomerengemische an. Die Isolierung der einzelnen geometrischen Isomeren erfolgt in üblicher Weise, wie z. B. aufgrund unterschiedlicher Löslichkeit, durch Salzbildung, durch Craig-Verteilung oder durch chemographische Trennverfahren bzw. durch Kombination dieser Methoden. Eine eindeutige Strukturzuordnung erfolgt aufgrund der ¹H-NMR-Daten, insbesondere unter Verwendung von Verschiebungsreagenzien.

Die Imidazolyl-Ketone der allgemeinen Formel II sind bekannt (vgl. DE-AS 2 105 490 und DE-OS 2 638 470) und können nach den dort angegebenen Verfahren erhalten werden, indem man z. B. entsprechende Halogenketone mit Imidazol in Gegenwart eines Verdünnungsmittels und in Gegenwart eines Säurebinders umsetzt.

Die außerdem für das erfindungsgemäße Verfahren als Ausgangsstoffe benötigten Alkylsulfate bzw. -halogenide sind allgemein bekannte Verbindungen der organischen Chemie.

Zur Herstellung von physiologisch verträglichen Säureadditionssalzen der Verbindungen der allgemeinen Formel I kommen vorzugsweise folgende Säuren in Frage: Die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Citronensäure, Salicylsäure, Sorbinsäure, Milchsäure, sowie Sulfonsäure, wie z. B. p-Toluolsulfonsäure und 1,5-Naphthalindisulfonsäure. Die Säureadditions-Salze der Verbindungen der allgemeinen Formel I können in einfacher Weise nach üblichen Salzbildungsmethoden, z. B. durch Lösen einer Verbindung der allgemeinen Formel I in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen in einem inerten organischen Lösungsmittel gereinigt werden.

Zur Herstellung von Metallsalz-Komplexen der Verbindungen der allgemeinen Formel I kommen vorzugsweise Salze von Metallen der II. bis IV. Haupt- und der I. und II. sowie IV. bis VIII. Nebengruppe in Frage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z. B. die Chlorwasserstoffsäure und die Bromwasserstoffsäure, ferner Phosphorsäure, Salpetersäure und Schwefelsäure.

Die Metallsalz-Komplexe von Verbindungen der allgemeinen Formel I können in einfacher Weise nach üblichen Verfahren erhalten werden, so z. B. durch Lösen des Metallsalzes in Alkohol, z. B. Ethanol und Hinzufügen zur Verbindung der allgemeinen Formel I. Man kann Metallsalz-Komplexe in bekannter Weise, z. B. durch Abfiltrieren, Isolieren und gegebenenfalls durch Umkristallisation reinigen.

Die erfindungsgemäß verwendbaren Verbindungen der allgemeinen Formel I, ihre Säureadditions-Salze und Metallsalz-Komplexe weisen antimikrobielle, insbesondere starke antimykotische Wirkungen auf. Sie besitzen ein sehr breites antimykotisches Wirkungsspektrum, insbesondere gegen Dermatophyten und Sproßpilze sowie biphasische Pilze, z. B. gegen Candida-Arten, wie Candida albicans, Epidermophyton-Arten, wie Epidermophyton floccosum, Aspergillus-Arten, wie Aspergillus niger und Aspergillus fumigatus, wie Trichophyton-Arten, wie Trichophyton mentagrophytes, Microsporon-Arten, wie Microsporon felineum sowie Penicillium-Arten, wie Penicillium commune. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden:

Dermatomykosen und Systemmykosen, durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiet in der Tiermedizin können beispielsweise aufgeführt werden:

Alle Dermatomykosen und Systemmykosen, insbesondere solche, die durch die obengenannten Erreger hervorgerufen werden.

Zur Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Zur Erfindung gehören auch pharmazeutische Zubereitungen in Dosierungseinheiten. Dies bedeutet, daß die Zubereitungen in Form einzelner Teile, z. B. Tabletten, Dragees, Kapseln, Pillen, Suppositorien durch Ampullen vorliegen, deren Wirkstoffgehalt einem Bruchteil oder einem Vielfachen einer Einzeldosis entsprechen. Die Dosierungseinheiten können z. B. 1, 2, 3 oder 4 Einzeldosen oder ½, ⅓ oder ¼ einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glucose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumbicarbonat, (e) Lösungsverzögerer, z. B. Paraffin und (f) Resorptionsbeschleuniger, z. B. quarternäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyäthylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z. B. Polymersubstanzen und Wachse verwendet werden können.

Der oder die Wirkstoffe können gegebenenfalls mit einem oder mehreren oder oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z. B. Polyäthylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel z. B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Suspensionen können neben dem oder den Wirkstoffen die üblichen Trägerstoffe wie flüssige Verdünnungsmittel, z. B. Wasser, Ethylalkohol, Propylenglykol, Suspendiermittel, z. B. äthoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z. B. Pfefferminzöl und Eukalyptusöl und Süßmittel, z. B. Sacharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise parenteral, insbesondere intravenös appliziert werden.

## Beispiel A

### Antimykotische in vitro-Wirksamkeit

### Versuchsbeschreibung

Die in-vitro-Prüfung wurde im Reihenverdünnungstest mit Keimimokula von durchschnittlich 5 × 10 Keimen/ml Substrat durchgeführt. Als Nährmedium dienten

a) für Dermatophyten und Schimmelpilze:
   Sabourand's milieu d'éspresive
b) für Hefen:
   Isotonic Sensitest-Broth von Oxid.

Die Bebrütungstemperatur betrug 18°C; Bebrütungszeit war 24 Stunden bei Hefen und 96 Stunden bei Dermatophyten und Schimmelpilzen.

In diesem Versuch zeigen die erfindungsgemäßen Verbindungen sehr gute MHK-Werte.

### Beispiel B

### Antimikrobielle in-vivo-Wirksamkeit (oral) bei Mäuse-Candidose

#### Versuchsbeschreibung

Mäuse vom Typ SPF-$CF_1$ wurden intravenös mit $1-2 \times 10^6$ logarithmisch wachsenden Candida-Zellen, die in physiologischer Kochsalzlösung suspendiert waren, infiziert. Eine Stunde vor und sieben Stunden nach der Infektion werden die Tiere mit jeweils 50—100 mg/kg Körpergewicht der Präparate oral behandelt.

#### Ergebnis

Unbehandelte Tiere starben 3 bis 6 Tage post infectionem. Die Überlebensrate am 6. Tag post infectionem betrug bei unbehandelten Kontrolltieren etwa 5%.

In diesem Versuch erweisen sich die erfindungsgemäßen Verbindungen als sehr gut wirksam.

### Beispiel C

### Antimikrobielle in-vivo-Wirksamkeit (lokal) am Modell der experimentellen Meerschweinchen-Trichophytie

#### Versuchsbeschreibung

Weiße Meerschweinchen der Rasse Pirbright-white wurden auf dem geschorenen, nicht skarifizierten Rücken mit einer Mikro- und Makrokonidien-Suspension von Trichophyton mentagrophytes infiziert.

Die infizierten Tiere wurden, beginnend mit dem 3. Tag p. i., 1× täglich mit 1%iger Lösung der erfindungsgemäßen Präparate (in Dimethylsulfoxid : Glycerin = 1 : 4) lokal behandelt.

#### Ergebnis

Bei unbehandelten Tieren entwickelt sich innerhalb von 12 Tagen p. i. das typische Bild einer Dermatophytose mit Rötung, Schuppung und Haarausfall bis zum totalen Integument-Defekt an der Infektionsstelle.

Dagegen zeigten z. B. die erfindungsgemäßen Beispiele 1 und 3 eine gute Wirkung, d. h. lediglich geringe Rötung und vereinzelt Schuppen.

## Herstellungsbeispiele

### Beispiel 1

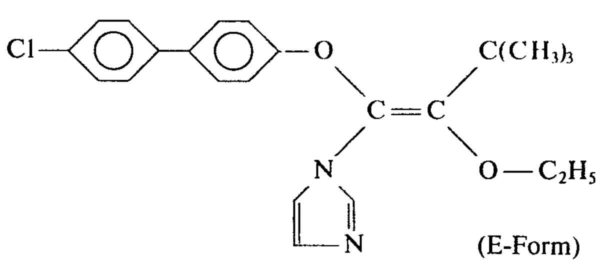

(E-Form)

Zu 36,85 g (0,1 Mol) 1-(4'-Chlor-4-biphenylyloxy)-3,3-dimethyl-1-(imidazol-1-yl)-butan-2-on in 50 ml Dimethylsulfoxid werden 6 g Kaliumhydroxid, in wenig Wasser gelöst, zugetropft. Man läßt kurze Zeit nachrühren und tropft anschließend 16 g (0,11 Mol) Diethylsulfat zu. Dabei wird die Temperatur der Reaktionsmischung auf ca. 40°C gehalten, danach ca. ½ Stunde auf 80°C. Man läßt abkühlen, versetzt mit Wasser, saugt den kristallinen Niederschlag ab und kristallisiert aus Petrolether um. Man erhält 22 g (50% der Theorie) (E)-1-(4'-Chlor-4-biphenylyloxy)-3,3-dimethyl-2-ethoxy-1-(imidazol-1-yl)-1-buten vom Schmelzpunkt 112—113°C.

In entsprechender Weise können die nachfolgenden Beispiele der allgemeinen Formel I

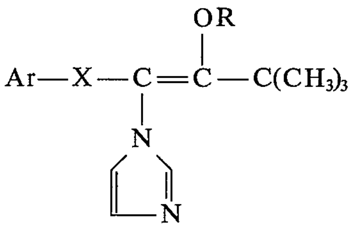

(I)

erhalten werden:

| Bsp. Nr. | Ar | X | R | Schmelzpunkt (°C) |
|---|---|---|---|---|
| 2 | 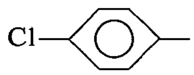 | O | $C_2H_5$ | 94–95 (E-Form) |
| 3 | 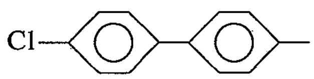 | O | $CH_3$ | 139–42 (E-Form) |

**Patentansprüche**

1. Antimykotisches Mittel, gekennzeichnet durch einen Gehalt an mindestens einem Imidazolylenolether der allgemeinen Formel I

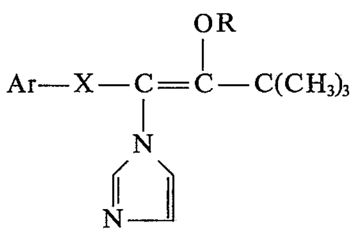

(I)

in welcher

Ar für gegebenenfalls einfach oder zweifach durch Halogen, Alkyl mit 1–3 Kohlenstoffatomen, Trifluormethyl, Nitro, Phenyl oder Chlorphenyl substituiertes Phenyl steht,
R für Alkyl mit 1–4 Kohlenstoffatomen steht und
X für Sauerstoff oder Methylen steht,

und/oder deren physiologisch verträglichen Säureadditions-Salze und/oder Metallsalz-Komplexe.

2. Antimykotisches Mittel nach Anspruch 1, gekennzeichnet durch einen Gehalt an 1-(4'-Chlor-4-biphenyloxy)-3,3-dimethyl-2-ethoxy-1-(imidazol-1-yl)-1-buten.

3. Antimykotisches Mittel nach Anspruch 1, gekennzeichnet durch einen Gehalt an 1-(4-Chlorphenoxy)-3,3-dimethyl-2-ethoxy-1-(imidazol-1-yl)-1-buten.

4. Antimykotisches Mittel nach Anspruch 1, gekennzeichnet durch einen Gehalt an 1-(4'-Chlor-4-biphenyloxy)-3,3-dimethyl-2-methoxy-1-(imidazol-1-yl)-1-buten.

5. Verfahren zur Herstellung eines antimykotischen Mittels, dadurch gekennzeichnet, daß man Imidazolylenolether gemäß der allgemeinen Formel I in Anspruch 1 mit inerten, nichttoxischen, pharmazeutisch geeigneten Trägerstoffen vermischt.

6. Verfahren zur Herstellung eines antimykotischen Mittels, dadurch gekennzeichnet, daß man den Imidazolylenolether gemäß Anspruch 2 mit inerten, nichttoxischen, pharmazeutisch geeigneten Trägerstoffen vermischt.

7. Verfahren zur Herstellung eines antimykotischen Mittels, dadurch gekennzeichnet, daß man den Imidazolylenolether gemäß Anspruch 3 mit inerten, nichttoxischen, pharmazeutisch geeigneten Trägerstoffen vermischt.

8. Verfahren zur Herstellung eines antimykotischen Mittels, dadurch gekennzeichnet, daß man den Imidazolylenolether gemäß Anspruch 4 mit inerten, nichttoxischen, pharmazeutisch geeigneten Trägerstoffen vermischt.

## Claims

1. Antimycotic agent, characterised in that it contains at least one imidazolyl-enol ether of the general formula I

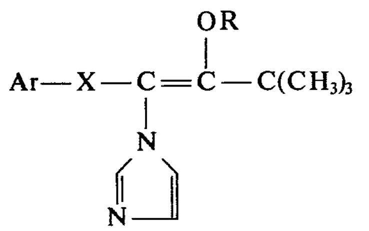

(I)

in which

Ar represents phenyl which is optionally substituted once or twice by halogen, alkyl having 1—3 carbon atoms, trifluoromethyl, nitro, phenyl or chlorophenyl,
R represents alkyl having 1—4 carbon atoms and
X represents oxygen or methylene,

and/or physiologically acceptable acid addition salts and/or metal salt complexes thereof.

2. Antimycotic agent according to Claim 1, characterised in that it contains 1-(4'-chloro-4-biphenyloxy)-3,3-dimethyl-2-ethoxy-1-(imidazol-1-yl)-1-butene.

3. Antimycotic agent according to Claim 1, characterised in that it contains 1-(4-chlorophenoxy)-3,3-dimethyl-2-ethoxy-1-(imidazol-1-yl)-1-butene.

4. Antimycotic agent according to Claim 1, characterised in that it contains 1-(4'-chloro-4-biphenyloxy)-3,3-dimethyl-2-methoxy-1-(imidazol-1-yl)-1-butene.

5. Process for the preparation of an antimycotic agent, characterised in that imidazolyl-enol ethers according to the general formula I in Claim 1 are mixed with inert, non-toxic, pharmaceutically suitable excipients.

6. Process for the preparation of an antimycotic agent, characterised in that the imidazolyl-enol ether according to Claim 2 is mixed with inert, non-toxic, pharmaceutically suitable excipients.

7. Process for the preparation of an antimycotic agent, characterised in that the imidazolyl-enol ether according to Claim 3 is mixed with inert, non-toxic, pharmaceutically suitable excipients.

8. Process for the preparation of an antimycotic agent, characterised in that the imidazolyl-enol ether according to Claim 4 is mixed with inert, non-toxic, pharmaceutically suitable excipients.

## Revendications

1. Composition antimycosique, caractérisée par une teneur en au moins un énol-éther d'imidazolyle de formule générale I:

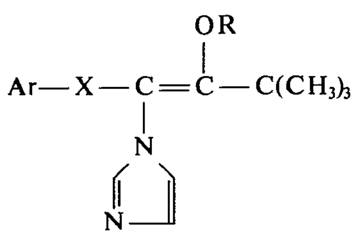

(I)

dans laquelle

Ar désigne un groupe phényle éventuellement substitué une fois ou deux fois par un halogène, un radical alkyle ayant 1 à 3 atomes de carbone, un radical trifluorométhyle, nitro, phényle ou chlorophényle,
R est un groupe alkyle ayant 1 à 4 atomes de carbone, et
X est l'oxygène ou le groupe méthylène,

et/ou leurs sels d'addition d'acides physiologiquement acceptables et/ou leurs complexes de sels métalliques.

2. Composition antimycosique suivant la revendication 1, caractérisée par une teneur en 1-(4'-chloro-4-biphényloxy)-3,3-diméthyl-2-éthoxy-1-(imidazol-1-yl)-1-butène.

3. Composition antimycosique suivant la revendication 1, caractérisée par une teneur en 1-(4-chlorophénoxy)-3,3-diméthyl-2-éthoxy-1-(imidazol-1-yl)-1-butène.

4. Composition antimycosique suivant la revendication 1, caractérisée par une teneur en 1-(4'-chloro-4-biphényloxy)-3,3-diméthyl-2-méthoxy-1-(imidazol-1-yl)-1-butène.

5. Procédé de production d'une composition antimycosique, caractérisé en ce qu'on mélange un énol-éther d'imidazolyle de formule générale I donnée dans la revendication 1 avec des supports inertes non toxiques acceptables du point de vue pharmaceutique.

6. Procédé de production d'une composition antimycosique, caractérisé en ce qu'on mélange l'énol-éther d'imidazolyle suivant la revendication 2 avec des supports inertes non toxiques pharmaceutiquement acceptables.

7. Procédé de production d'une composition antimycosique, caractérisé en ce qu'on mélange l'énol-éther d'imidazolyle suivant la revendication 3 avec des supports inertes non toxiques acceptables du point de vue pharmaceutique.

8. Procédé de production d'une composition antimycosique, caractérisé en ce qu'on mélange l'énol-éther d'imidazolyle suivant la revendication 4 avec des supports inertes non toxiques acceptables du point de vue pharmaceutique.